# EUROPEAN PATENT APPLICATION

(11) **EP 4 691 645 A1**
(43) Date of publication of application: **11.02.2026**
(21) Application number: 24823459.3
(22) Date of filing: 14.06.2024
(51) Int. Cl.: B04B 5/02, A61M 1/02

(54) **CENTRIFUGAL SEPARATION SYSTEM, ROTATING BODY, AND CENTRIFUGAL SEPARATOR**

(30) Priority: 16.06.2023 JP 2023099358
(71) Applicant: Terumo Kabushiki Kaisha, Tokyo 151-0072 (JP)
(72) Inventor: KATAOKA, Ryoji, Fujinomiya-shi, Shizuoka 418-0004 (JP)
(74) Representative: KIPA AB
(86) International application number: PCT/JP2024/021610
(87) International publication number: WO 2024/257846

(57) **Abstract**

A centrifugal separation system (10) performs centrifugal separation using a centrifugal bag set (16) including a first bag (161) that accommodates a liquid to be processed, and one or a plurality of child bags that exchanges the liquid with the first bag (161). The centrifugal separation system (10) includes a rotating body (14) that accommodates one centrifugal bag set (16), and a centrifugal separator (12) that rotates the rotating body (14). The rotating body (14) is attached to and detached from the centrifugal separator (12) in a state of accommodating the centrifugal bag set (16).

## Description

### Technical Field

The present invention relates to a centrifugal separation system, a rotating body, and a centrifugal separator that separate a mixed liquid into a plurality of components using a centrifugal bag set.

### Background Art

For example, Japanese Patent No. 5272144 discloses a centrifugal separator that separates a liquid using a centrifugal bag set. This centrifugal separator includes a plurality of slots for accommodating a plurality of centrifugal bag sets in a rotatable rotating body.

### Citation List

### Patent Literature

Patent Literature 1: Japanese Patent No. 5272144

### Summary of Invention

A conventional centrifugal separation system is a dedicated device corresponding to a type of a centrifugal bag set, and it is necessary to prepare a new centrifugal separator in a case of processing different types of centrifugal bag sets.

An object of the present invention is to solve the problem described above.

One aspect is (1) a centrifugal separation system that performs centrifugal separation using a centrifugal bag set including a first bag that accommodates a liquid to be processed, one or a plurality of child bags that exchanges the liquid with the first bag, and at least one tube that connects the first bag to the child bag, the centrifugal separation system including a rotating body that accommodates the one centrifugal bag set, and a centrifugal separator that rotates the rotating body, in which the rotating body is attached to and detached from the centrifugal separator that centrifugally separates the centrifugal bag set in a state in which the centrifugal bag set is accommodated.

In the centrifugal separation system according to the above-described item (1), the centrifugal separator can be used for processing a plurality of types of centrifugal bag sets by replacing the rotating body. Therefore, the centrifugal separation system can increase utilization efficiency of the centrifugal separator, and can flexibly respond to the demand.

(2) The centrifugal separation system according to the item (1), in which the rotating body may include a plurality of slots that is arranged to be spaced apart from each other in a circumferential direction of a rotation axis, and accommodates the first bag and the child bag to be spaced apart from each other in the circumferential direction, and a liquid delivery mechanism that transfers the liquid between the first bag and the child bag spaced apart from each other in the circumferential direction. This centrifugal separation system can transfer blood components between a plurality of bags while reducing a size of the rotating body, so that manual work can be reduced.

(3) The centrifugal separation system according to the item (1) or (2), in which the rotating body may include a balancer that cancels a displacement in gravity center position of the centrifugal bag set. This centrifugal separation system can stabilize rotational motion of the rotating body.

(4) The centrifugal separation system according to the item (2), in which the liquid delivery mechanism may include an extrusion unit that presses the first bag or the child bag in at least one of the slots. This centrifugal separation system can automate the transfer of the liquid between the bags.

(5) The centrifugal separation system according to the item (4), in which the liquid delivery mechanism may include a tube accommodation unit that accommodates the tube, and a close cut unit that is disposed in the tube accommodation unit and is capable of closing or sealing the tube. This centrifugal separation system makes it possible to separate different components into different child bags. This centrifugal separation system can automate a work of sealing the tube connected to the child bag.

(6) The centrifugal separation system according to the item (5), in which the rotating body may include a liquid sensor that detects a component of the liquid inside the tube. This centrifugal separation system can automate separation of the blood components.

(7) The centrifugal separation system according to any one of the items (1) to (6), in which the rotating body may include a reading device that reads identification information from the centrifugal bag set. This centrifugal separation system can save labor for an input operation of the identification information of the centrifugal bag set.

(8) The centrifugal separation system according to the item (7), in which the rotating body may include a first control unit that receives a control signal of the centrifugal separator and transmits the identification information of the centrifugal bag set to the centrifugal separator. This centrifugal separation system can automate the input operation of the identification information of the centrifugal bag set while simplifying the configuration of the first control unit of the rotating body and saving the labor.

(9) The centrifugal separation system according to the item (8), in which the first control unit may drive the liquid delivery mechanism on the basis of the control signal. In this centrifugal separation system, the configuration of the first control unit can be simplified, and the rotating body can be further downsized easily.

(10) The centrifugal separation system according to the item (8) or (9), in which the centrifugal separator can include a rotating body accommodation chamber that accommodates the rotating body, a rotational drive unit that rotates the rotating body, and a second control unit that controls the rotational drive unit, and the second control unit can monitor the rotating body and deliver the control signal to the first control unit. The centrifugal separation system can more efficiently perform the centrifugal separation of the centrifugal bag set by monitoring the rotating body.

(11) The centrifugal separation system according to any one of the items (1) to (10), in which the rotating body may include a temperature measurement unit disposed in each of the plurality of slots, and the temperature measurement unit may measure temperatures of the first bag and the child bag. This centrifugal separation system makes it possible to confirm that the blood has been formulated in a predetermined temperature range.

Another aspect is (12) a rotating body of a centrifugal separation system that performs centrifugal separation using one centrifugal bag set including a first bag that accommodates a liquid to be processed, one or a plurality of child bags that exchanges the liquid with the first bag, and a tube that connects the first bag to the child bag, the rotating body including a first slot that accommodates the first bag, at least one second slot that is arranged to be spaced apart from the first slot in a circumferential direction of a rotation axis and accommodates the child bag, and a liquid delivery mechanism that transfers the liquid between the first bag of the first slot and the child bag of the second slot, the rotating body being attached to and detached from a centrifugal separator that centrifugally separates the centrifugal bag set in a state in which the centrifugal bag set is accommodated. Since this rotating body accommodates one centrifugal bag set, this can be downsized. Since the rotating body can attach and detach the centrifugal bag set in a place different from the centrifugal separator, a standby time of the centrifugal separator can be reduced, and an operating rate of the centrifugal separator can be increased.

(13) The rotating body according to the item (12), further including a temperature measurement unit disposed in each of the first slot and the second slot, in which the temperature measurement unit may measure temperatures of the first bag and the child bag. This rotating body enables confirmation that the blood has been formulated in a predetermined temperature range.

Still another aspect is (14) a centrifugal separator of a centrifugal separation system that performs centrifugal separation using one centrifugal bag set including a first bag that accommodates a liquid to be processed, one or a plurality of child bags that exchanges the liquid with the first bag, and a tube that connects the first bag to the child bag, the centrifugal separator including a rotational drive unit that supports a rotating body that accommodates the centrifugal bag set so as to be attachable and detachable and rotates the rotating body, and a second control unit that delivers a control signal to the rotating body, in which the second control unit monitors an operation of the rotating body. This centrifugal separator can downsize a device configuration along with the downsizing of the rotating body. Since the centrifugal separator can attach and detach the centrifugal bag set to and from the rotating body in another place, the operating rate can be increased by reducing the standby time of the device.

The centrifugal separation system, the rotating body, and the centrifugal separator described above have excellent utilization efficiency and can flexibly respond to demand.

### Brief Description of Drawings

Fig. 1 is a perspective view of a centrifugal separator and a rotating body of a centrifugal separation system according to a first embodiment.
Fig. 2A is a perspective view of the rotating body in Fig. 1, and Fig. 2B is a plan view of the rotating body in Fig. 1.
Fig. 3 is a system configuration diagram of the rotating body in Fig. 1.
Fig. 4 is a system configuration diagram of the centrifugal separator in Fig. 1.
Fig. 5A is an explanatory view of a centrifugal bag set according to the first embodiment and a whole blood sampling method using the centrifugal bag set, and Fig. 5B is an explanatory view of a step of removing white blood cells and platelets from the whole blood using the centrifugal bag set in Fig. 5A and a step of separating the blood sampling bag.
Fig. 6A is an explanatory view of a state in which the centrifugal bag set in Fig. 5B is attached to the rotating body in Fig. 2B, and Fig. 6B is an explanatory view of a step of transferring blood components centrifugally separated in the first bag using the rotating body to the second bag (child bag).
Fig. 7A is an explanatory view of a step of transferring a preservative solution in a third bag (child bag) to the blood components in the first bag using the rotating body, and Fig. 7B is an explanatory view of a step of closing and sealing a tube.
Fig. 8A is a plan view of a rotating body according to a second embodiment, and Fig. 8B is an explanatory view illustrating a system configuration of the rotating body in Fig. 8A and a configuration of a centrifugal bag set according to the second embodiment.
Fig. 9A is an explanatory view of a blood sampling step using the centrifugal bag set in Fig. 8B, and Fig. 9B is an explanatory view of a centrifugal separation step using the rotating body in Fig. 8A.
Fig 10A is an explanatory view of a step of transferring blood components to a second bag (child bag) using the rotating body in Fig. 8A, and Fig. 10B is an explanatory view of a step of transferring the blood components to a third bag (child bag) using the rotating body in Fig. 8A.
Fig. 11A is an explanatory view of a step of transferring a preservative solution in a fourth bag to a first bag, and Fig. 11B is an explanatory view of a step of transferring a mixed solution of the preservative solution and blood components in the first bag to the fourth bag.
Fig. 12A is a perspective view of a rotating body according to a third embodiment, and Fig. 12B is a plan view of the rotating body in Fig. 12A.

### Description of Embodiments

### (First Embodiment)

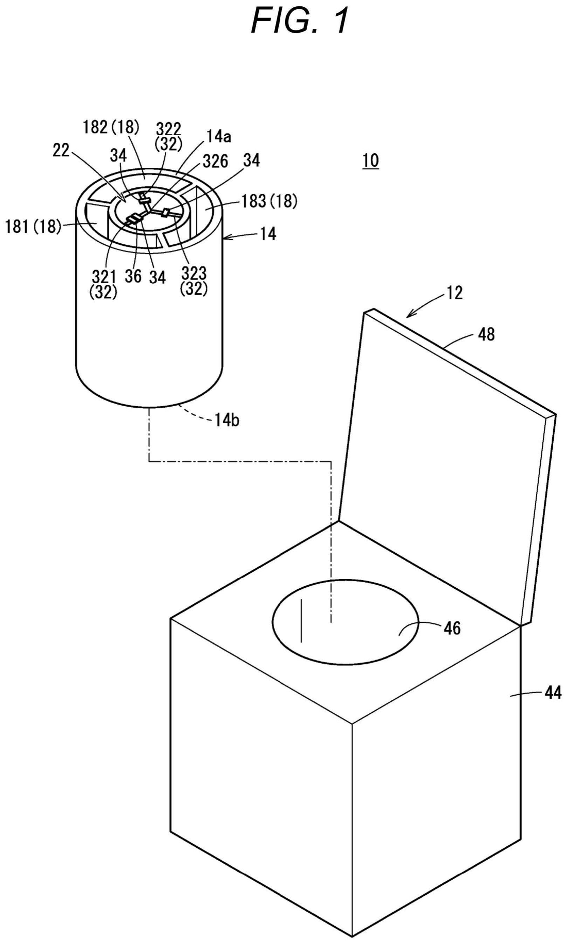

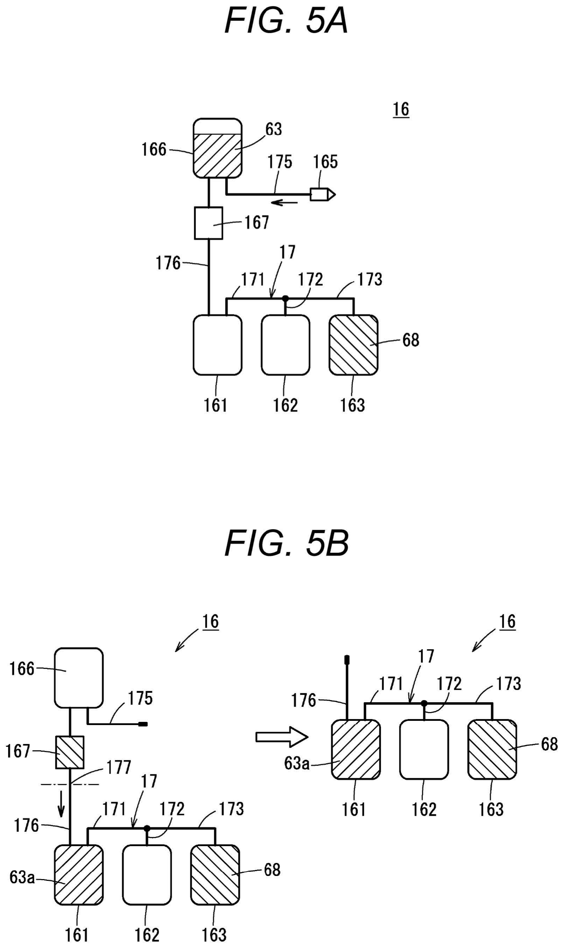

As illustrated in Fig. 1, a centrifugal separation system 10 according to the present embodiment includes a centrifugal separator 12 and a rotating body 14. The rotating body 14 is attachable to and detachable from the centrifugal separator 12. When the rotating body 14 is attached to the centrifugal separator 12, the centrifugal separator 12 enables rotational motion about a rotation axis. The rotating body 14 accommodates a centrifugal bag set 16 illustrated in Fig. 5A, and applies a centrifugal force to the centrifugal bag set 16 to separate a liquid to be processed into a plurality of components. The centrifugal separation system 10 is suitably used, for example, in a blood center. In this case, the centrifugal separation system 10 centrifugally separates blood (whole blood 63) as a liquid to formulate red blood cells 64 and plasma 66. Note that, the use of the centrifugal separation system 10 is not limited to the separation of the blood, and this can also be used for purification of cell medicines and formulation of cell products.

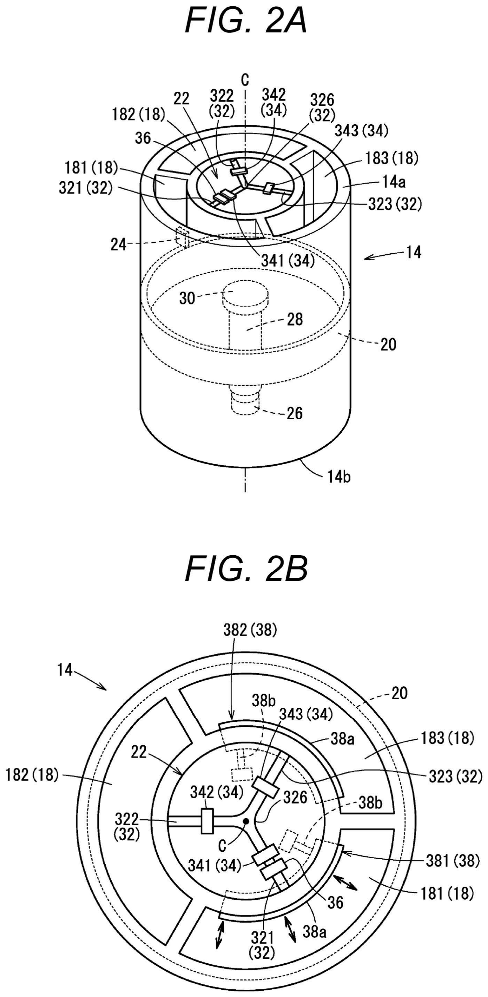

As illustrated in Figs. 2A and 2B, the rotating body 14 has a columnar shape centered on a rotation axis C, includes a flat upper end face 14a at an upper end, and includes a flat lower end face 14b at a lower end. The rotating body 14 includes a plurality of slots 18 (first slot 181, second slot 182, and third slot 183), a balancer 20, a liquid delivery mechanism 22, a reading device 24, an attaching/detaching unit 26, a power supply device 28, and a first control unit 30.

The first slot 181, the second slot 182, and the third slot 183 are formed as recesses recessed in a rotation axis direction with respect to the upper end face 14a, and open to the upper end face 14a. The first slot 181 accommodates a first bag 161 of the centrifugal bag set 16. The second slot 182 accommodates a second bag 162 of the centrifugal bag set 16. The third slot 183 accommodates a third bag 163 of the centrifugal bag set 16.

The rotating body 14 includes the same number of slots 18 (first slot 181 to third slot 183) as the bags (first bag 161 to third bag 163) of the centrifugal bag set 16. The number of slots of the rotating body 14 can be increased or decreased according to the number of bags of the centrifugal bag set 16. The first slot 181, the second slot 182, and the third slot 183 are arranged to be spaced apart from each other at equal intervals in a circumferential direction around the rotation axis C.

The balancer 20 is an annular member arranged near an outer peripheral surface of the rotating body 14. The balancer 20 is also called a balance ring, and includes an internal space extending in the circumferential direction and a balancer liquid flowing in the internal space. The balancer 20 stabilizes the rotational motion of the rotating body 14 by cancelling displacement in gravity center position due to displacement of an arrangement position of the centrifugal bag set 16 in the slot 18. The balancer 20 accommodates the balancer liquid of an amount that can cancel the displacement in the gravity center position caused by liquid exchange between the first bag 161 and the second or third bag 162 or 163.

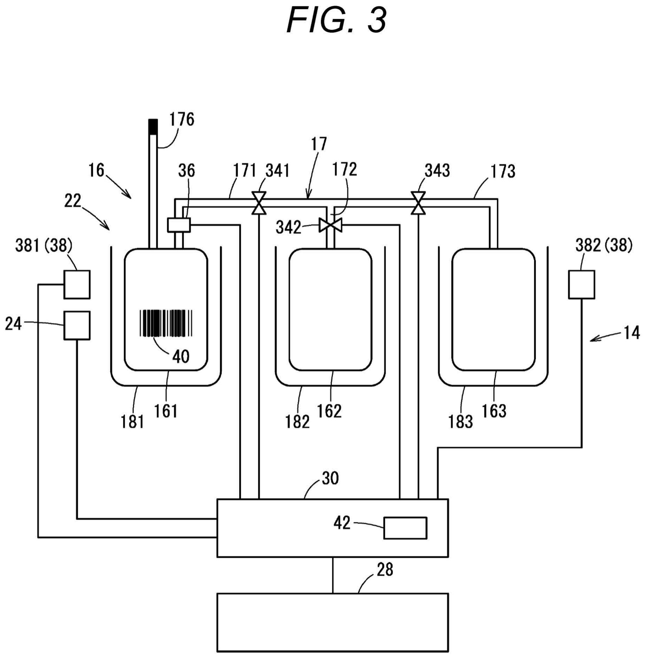

The liquid delivery mechanism 22 moves the liquid between a plurality of bags of the centrifugal bag set 16. The liquid delivery mechanism 22 mainly includes a tube accommodation unit 32, a close cut unit 34, a liquid sensor 36, and an extrusion unit 38. The tube accommodation unit 32 is located on the upper end face 14a of the rotating body 14. The tube accommodation unit 32 includes a first flow path groove 321, a second flow path groove 322, and a third flow path groove 323. The first flow path groove 321 is a groove-shaped portion that accommodates a first tube 171 (refer to Fig. 3) extending from the first bag 161.
The second flow path groove 322 is a groove-shaped portion that accommodates a second tube 172 extending from the second bag 162. The third flow path groove 323 is a groove-shaped portion that accommodates a third tube 173 extending from the third bag 163. The first flow path groove 321, the second flow path groove 322, and the third flow path groove 323 are connected at a merging portion 326. Note that, a layout of the first flow path groove 321, the second flow path groove 322, and the third flow path groove 323 is not limited to the illustrated example, and can have various shapes.

The liquid delivery mechanism 22 includes, as the close cut unit 34, a first close cut unit 341 disposed in the first flow path groove 321, a second close cut unit 342 disposed in the second flow path groove 322, and a third close cut unit 343 disposed in the third flow path groove 323. Each close cut unit 34 performs a closing operation of compressing to close the tube 17 and a seal cutting operation of cutting the tube 17 while sealing the same. Note that, in the present specification, a plurality of connection tubes included in the centrifugal bag set 16 is collectively referred to as the tubes 17. In the closing operation, the close cut unit 34 compresses the tube 17 to such an extent as not to cut the tube 17, thereby closing the flow path inside the tube 17. In the closing operation, the tube 17 is only temporarily closed. Therefore, when the closing operation of the close cut unit 34 is released, the tube 17 can allow fluid to flow. In the seal cutting operation, the close cut unit 34 cuts the tube 17 while heating the tube 17 and irreversibly sealing the same by fusion. By the seal cutting operation, the tube 17 is sealed without coming into contact with outside air.

The liquid sensor 36 is, for example, an optical sensor, and is located in the first flow path groove 321. The liquid sensor 36 detects a change in liquid components inside the first tube 171 from a change in amount or color of light transmitted through the first tube 171. A detection result of the liquid sensor 36 is delivered to the first control unit 30, and is used to control the operations of the close cut unit 34 and the extrusion unit 38.

The extrusion unit 38 moves the liquid between the bags by pressing a predetermined bag of the centrifugal bag set 16. The extrusion unit 38 of the present embodiment includes a first extrusion unit 381 disposed in the first slot 181 and a second extrusion unit 382 disposed in the third slot 183. Each of the first extrusion unit 381 and the second extrusion unit 382 includes a pusher 38a and an actuator 38b. The pusher 38a is a plate-shaped member arranged on an inner peripheral side of the slot 18. The pusher 38a can protrude from the inner peripheral side to an outer peripheral side of the slot 18. The pusher 38a faces the bag accommodated inside the slot 18 in a thickness direction. When the pusher 38a protrudes to the outer peripheral side, this presses the bag accommodated in the slot 18 to discharge the liquid inside the bag. The actuator 38b drives the pusher 38a under the control of the first control unit 30. The actuator 38b extrudes the pusher 38a toward the outer peripheral side. The first extrusion unit 381 transfers the liquid in the first bag 161 accommodated in the first slot 181 to another bag, and the second extrusion unit 382 transfers the liquid in the third bag 163 accommodated in the third slot 183 to another bag.

Note that, the extrusion unit 38 can be replaced with a peristaltic pump. The peristaltic pump is a pump that conveys the liquid inside the tube 17 by a roller rotating a portion closed by pressing the flexible tube 17 moving the liquid. In a case of employing the peristaltic pump, the peristaltic pump is added to the tube accommodation unit 32.

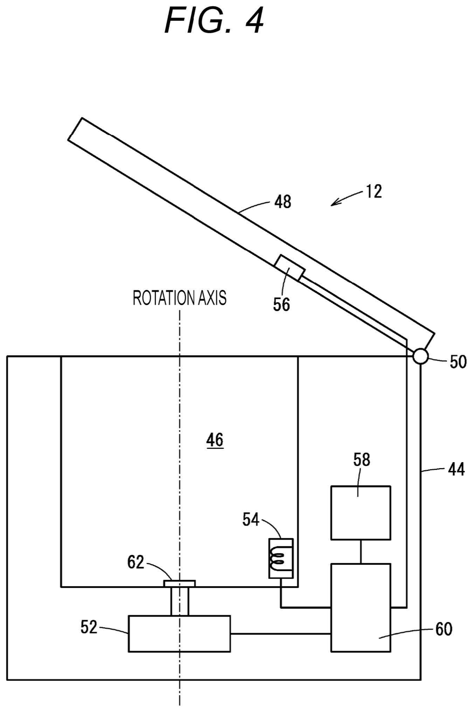

The attaching/detaching unit 26 is located on the lower end face 14b of the rotating body 14. The attaching/detaching unit 26 is arranged along the rotation axis. The attaching/detaching unit 26 is detachably connected to a rotation shaft 62 (refer to Fig. 4) of the centrifugal separator 12. A rotational driving force of the centrifugal separator 12 is transmitted to the rotating body 14 through the attaching/detaching unit 26.

The reading device 24 is arranged inside the first slot 181, for example. The reading device 24 may be an optical reading device that reads a barcode 40 attached to the first bag 161. The reading device 24 reads a type of the centrifugal bag set 16, traceability information such as blood sampling date and time and place, and identification information including a processing method. The identification information is used to control the operation of the liquid delivery mechanism 22 at a centrifugal separation step. Note that, the identification information may be stored in a non-contact IC chip attached to the centrifugal bag set 16, and in this case, the reading device 24 can be arranged at a predetermined site of the rotating body 14 as a wireless reading device.

As illustrated in Fig. 3, the power supply device 28 supplies drive power to the liquid delivery mechanism 22, the reading device 24, and the first control unit 30. The power supply device 28 includes, for example, a storage battery such as a lithium ion battery accommodated inside the rotating body 14. The power supply device 28 may be a power supply circuit that generates electric power by an electromagnetic field supplied from the centrifugal separator 12. The power supply device 28 may be a power supply rectifier circuit that is disposed in the attaching/detaching unit 26 or another portion of the rotating body 14 and rectifies the electric power from the centrifugal separator 12, a voltage conversion circuit that converts a voltage from the centrifugal separator 12 and the like.

As illustrated in Fig. 3, the first control unit 30 may include a first communication unit 42 that communicates with the centrifugal separator 12. The first control unit 30 transmits the identification information read by the reading device 24 and the detection result of the liquid sensor 36 to the centrifugal separator 12 via the first communication unit 42. The first control unit 30 receives a control signal of the centrifugal separator 12 via the first communication unit 42. The first control unit 30 drives the first extrusion unit 381, the second extrusion unit 382, the first close cut unit 341, the second close cut unit 342, and the third close cut unit 343 on the basis of the control signal of the centrifugal separator 12. Note that, the first communication unit 42 communicates with the centrifugal separator 12 in a wired or wireless manner.

As illustrated in Figs. 1 and 4, the centrifugal separator 12 includes a main body 44, a rotating body accommodation chamber 46, and a lid 48. The rotating body accommodation chamber 46 is a columnar recess formed inside the main body 44 and accommodates the rotating body 14. The lid 48 is attached to the main body 44 via a hinge 50 (Fig. 4). The lid 48 covers the rotating body 14 accommodated in the rotating body accommodation chamber 46 to protect the periphery from the rotating body 14 that rotates. The lid 48 stabilizes temperature inside the rotating body accommodation chamber 46 by covering the rotating body accommodation chamber 46.

The centrifugal separator 12 includes a motor 52 (rotational drive unit), a temperature regulator 54, a monitoring unit 56, a second communication unit 58, and a second control unit 60 inside the main body 44. The motor 52 is located below the rotating body accommodation chamber 46. The motor 52 includes a rotation shaft 62 extending along the rotation axis. An upper end of the rotation shaft 62 protrudes into the rotating body accommodation chamber 46. The rotation shaft 62 supports the attaching/detaching unit 26 of the rotating body 14 so as to be attachable and detachable. The motor 52 rotates the rotating body 14 at a predetermined rotation speed under the control of the second control unit 60. Although not particularly limited, the rotation shaft 62 may include an electrode terminal that supplies electric power to the power supply device 28 of the rotating body 14.

The temperature regulator 54 includes, for example, a heater, and maintains the inside of the rotating body accommodation chamber 46 at predetermined temperature. The temperature regulator 54 is driven by the second control unit 60.

The monitoring unit 56 includes an image sensor that monitors an internal state of the centrifugal bag set 16. The monitoring unit 56 monitors the inside of the first bag 161 and detects a separation state of the liquid components.

The second communication unit 58 communicates with the first communication unit 42 of the rotating body 14. The second communication unit 58 receives the identification information acquired by the first control unit 30 and the detection result of the liquid sensor 36. The second communication unit 58 transmits a control signal of the second control unit 60 to the first control unit 30.

The second control unit 60 acquires the identification information of the centrifugal bag set 16 accommodated in the rotating body 14 through the second communication unit 58. The second control unit 60 sets the rotation speed of the motor 52 on the basis of the processing method out of the identification information. The second control unit 60 selects a control program on the basis of the processing method and the detection result of the liquid sensor 36. The second control unit 60 controls the operations of the extrusion unit 38 and the close cut unit 34 according to the selected control program. Note that, the control program corresponding to the processing method and the detection result of the liquid sensor 36 is stored in a memory not illustrated.

The rotating body 14 and the centrifugal separator 12 of the present embodiment are configured as described above, but the present embodiment is not limited thereto. For example, the first control unit 30 of the rotating body 14 may perform a part of the operation of the second control unit 60 of the centrifugal separator 12.

Next, an action of the centrifugal separation system 10 of the present embodiment will be described by taking processing of the blood using the centrifugal bag set 16 as an example. The centrifugal bag set 16 illustrated in Fig. 5A is, for example, a blood bag set used for blood donation. As illustrated in Fig. 5A, the centrifugal bag set 16 includes a blood sampling needle 165, a blood sampling bag 166, a filter 167, a first bag 161 (parent bag), a second bag 162 (child bag), and a third bag 163 (child bag) in an initial state of being provided as a product.

The blood sampling needle 165 includes a needle tube that punctures a donor, and samples the blood from the donor. The blood sampling bag 166 is connected to the blood sampling needle 165 through a blood sampling tube 175. The blood sampling bag 166 accommodates the blood (whole blood 63) of the donor sampled with the blood sampling needle 165. One end of an introduction tube 176 is connected to the blood sampling bag 166. The introduction tube 176 connects the blood sampling bag 166 and the first bag 161. The first bag 161 is connected to the blood sampling bag 166 via the introduction tube 176. The filter 167 is attached to the middle of the introduction tube 176.

The filter 167 adsorbs and removes white blood cells and platelets from the blood flowing through the introduction tube 176. The first bag 161 (parent bag) accommodates the blood flowing in from the introduction tube 176. In the initial state, the first bag 161 is empty. The first bag 161 in the initial state may contain an additive that prevents deterioration of the blood as necessary. As illustrated in Fig. 3, a barcode 40 (refer to Fig. 3) is attached to the first bag 161.

The first bag 161 further includes a first tube 171 separately from the introduction tube 176. The first tube 171 is used to exchange the liquid with the second bag 162 or the third bag 163. The second bag 162 is empty in the initial state. The second bag 162 accommodates blood components 63a (for example, plasma 66) centrifugally separated in the first bag 161. The second bag 162 includes the second tube 172. The second tube 172 is connected to the first tube 171.

The third bag 163 is, for example, the child bag accommodating a preservative solution 68. The preservative solution 68 is, for example, a blood preservative solution (ACD solution) or a red blood cell preservative solution (MAP solution). The third bag 163 includes a third tube 173. The third tube 173 is connected to the first tube 171 and the second tube 172.

As illustrated in Fig. 5A, in the centrifugal bag set 16, the whole blood 63 of the donor is sampled in the blood sampling bag 166 through the blood sampling needle 165. Thereafter, the blood sampling tube 175 is cut. The blood sampling needle 165 is separated from the centrifugal bag set 16.

Next, an operation of transferring the whole blood 63 in the blood sampling bag 166 to the first bag 161 through the introduction tube 176 is performed. In this operation, the white blood cells and platelets are removed from the whole blood 63 by the filter 167.

Next, as illustrated in Fig. 5B, an operation of cutting the introduction tube 176 at a cutting position 177 is performed. The centrifugal bag set 16 from which the filter 167 and the blood sampling bag 166 are removed, as the right centrifugal bag set 16 in Fig. 5B, is used for processing of the centrifugal separation system 10.

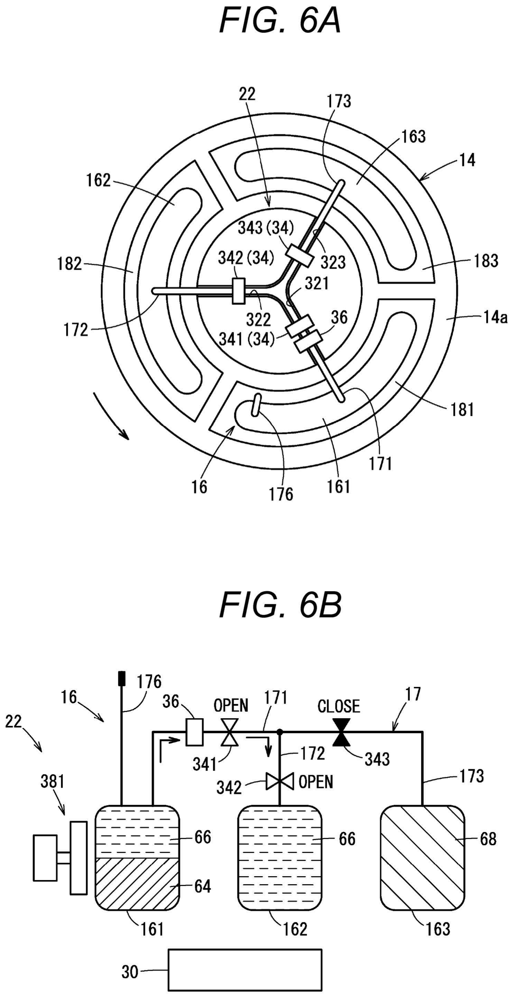

Next, an operation of accommodating the centrifugal bag set 16 in the rotating body 14 is performed. As illustrated in Fig. 6A, the first bag 161 is accommodated in the first slot 181, the second bag 162 is accommodated in the second slot 182, and the third bag 163 is accommodated in the third slot 183. The first tube 171 is accommodated in the first flow path groove 321, the second tube 172 is accommodated in the second flow path groove 322, and the third tube 173 is accommodated in the third flow path groove 323. The liquid sensor 36 and the close cut unit 34 are attached to the first tube 171. The close cut unit 34 is also attached to each of the second tube 172 and the third tube 173. The centrifugal bag set 16 can be attached to the rotating body 14 in a state in which the rotating body 14 is detached from the centrifugal separator 12. An attaching/detaching operation of the centrifugal bag set 16 to/from the rotating body 14 requires a predetermined time. By performing the attaching/detaching operation of the centrifugal bag set 16 to/from the rotating body 14 while detaching the same from the centrifugal separator 12, a standby time of the centrifugal separator 12 can be reduced, and an operating rate of the centrifugal separator 12 is improved.

In the centrifugal separation system 10, by sequentially mounting the rotating body 14 in which the centrifugal bag set 16 is accommodated to the centrifugal separator 12 to perform the centrifugal separation, a standby time until a plurality of centrifugal bag sets 16 is collected becomes unnecessary, so that the operating rate of the centrifugal separator 12 can be increased.

Next, as illustrated in Fig. 6B, a centrifugal separation step using the centrifugal separator 12 is performed. At a first step of the centrifugal separation step, the centrifugal separator 12 rotates the rotating body 14 at a predetermined rotation speed. By the rotation of the rotating body 14, the blood components 63a in the first bag 161 are separated according to a specific gravity. As illustrated, the blood in the first bag 161 is separated into the red blood cells 64 having a large specific gravity and a supernatant (plasma 66) having a small specific gravity. The plasma 66 is formed at a position close to the first tube 171 of the first bag 161.

Thereafter, a second step of transferring the plasma 66 in the first bag 161 to the second bag 162 is performed. As illustrated, at the second step, the first close cut unit 341 opens the first tube 171, the second close cut unit 342 opens the second tube 172, and the third close cut unit 343 closes the third tube 173. The first close cut unit 341 and the second close cut unit 342 are opened. As a result, the first bag 161 and the second bag 162 communicate with each other via the first tube 171 and the second tube 172. In this state, an operation of pressing the first bag 161 by the first extrusion unit 381 is performed. By this operation, the plasma 66 in the first bag 161 flows through the first tube 171 and the second tube 172 and is transferred to the second bag 162. The second step is performed under the monitoring of the liquid sensor 36. When the red blood cell 64 flows into the first tube 171, the liquid sensor 36 detects a change in the liquid components. When the change in the components in the first tube 171 is detected, the first control unit 30 closes the second close cut unit 342 of the second tube 172 to prevent the inflow of the red blood cells 64 into the second bag 162.

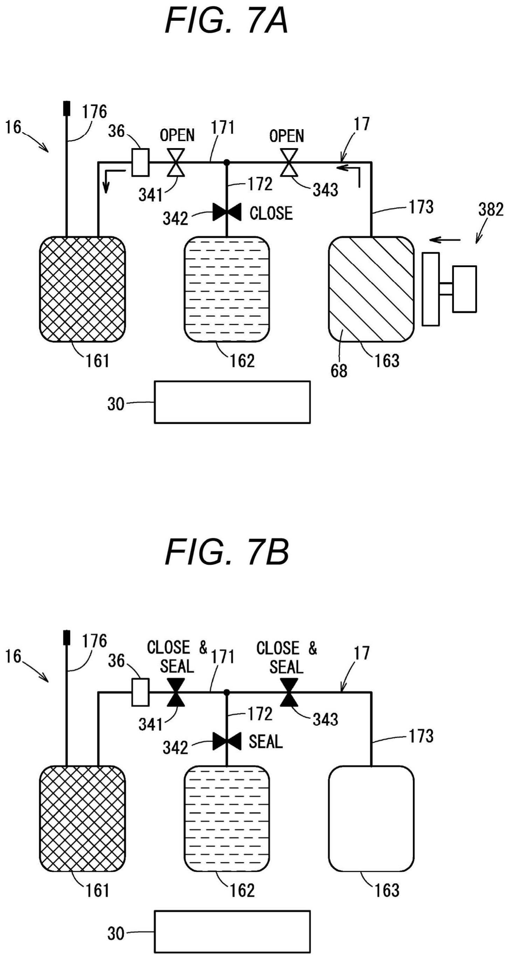

Next, as illustrated in Fig. 7A, a third step of transferring the preservative solution 68 in the third bag 163 to the first bag 161 is performed. At the third step, the first close cut unit 341 opens the first tube 171, the second close cut unit 342 closes the second tube 172, and the third close cut unit 343 opens the third tube 173. Furthermore, the first control unit 30 drives the second extrusion unit 382 to press the third bag 163. The preservative solution 68 in the third bag 163 is transferred to the first bag 161. When the second extrusion unit 382 completely presses the third bag 163, the third step is finished. By the above-described steps, the separation of the blood components 63a and the addition of the preservative solution 68 are completed.

Thereafter, a fourth step of sealing and cutting the tube 17 is performed as illustrated in Fig. 7B. The first close cut unit 341 seals and cuts the first tube 171, the second close cut unit 342 seals and cuts the second tube 172, and the third close cut unit 343 seals and cuts the third tube 173. At the fourth step, the first bag 161 is separated from the second bag 162 and the third bag 163. The second bag 162 is separated from the third bag 163 by sealing and cutting the second tube 172.

By the above-described steps, a centrifugal separation operation in the centrifugal separator 12 is completed. Thereafter, the rotating body 14 is detached from the centrifugal separator 12. A next unprocessed rotating body 14 is attached to the centrifugal separator 12 from which the rotating body 14 is detached, and a next centrifugal separation operation is performed. The rotating body 14 detached from the centrifugal separator 12 is conveyed to the outside of the centrifugal separator 12. Thereafter, the first bag 161, the second bag 162, and the third bag 163 are detached from the rotating body 14.

As described above, in the centrifugal separation system 10 of the present embodiment, since the rotating body 14 can be downsized, the centrifugal separator 12 can be downsized. Since the centrifugal bag set 16 can be attached to and detached from the rotating body 14 in a place different from the centrifugal separator 12, the centrifugal separation system 10 can reduce the standby time of the centrifugal separator 12 and increase the operating rate of the centrifugal separator 12. Furthermore, the rotating body 14 of the present embodiment can automatically transfer the centrifugally separated blood components 63a to the second bag 162, add the preservative solution 68 in the third bag 163 to the first bag 161, and seal and separate the first bag 161 and the second bag 162. Therefore, the centrifugal separation system 10 can save labor.

### (Second Embodiment)

The present embodiment relates to a modification of a rotating body 14A and a centrifugal bag set 16A. Note that, since the configuration of the centrifugal separator 12 is the same, the description of the centrifugal separator 12 is omitted in the present embodiment. In the configurations of the rotating body 14A and the centrifugal bag set 16A of the present embodiment, the same reference numerals are given to the configurations similar to those described with reference to Figs. 1 to 7B, and the detailed description thereof will be omitted.

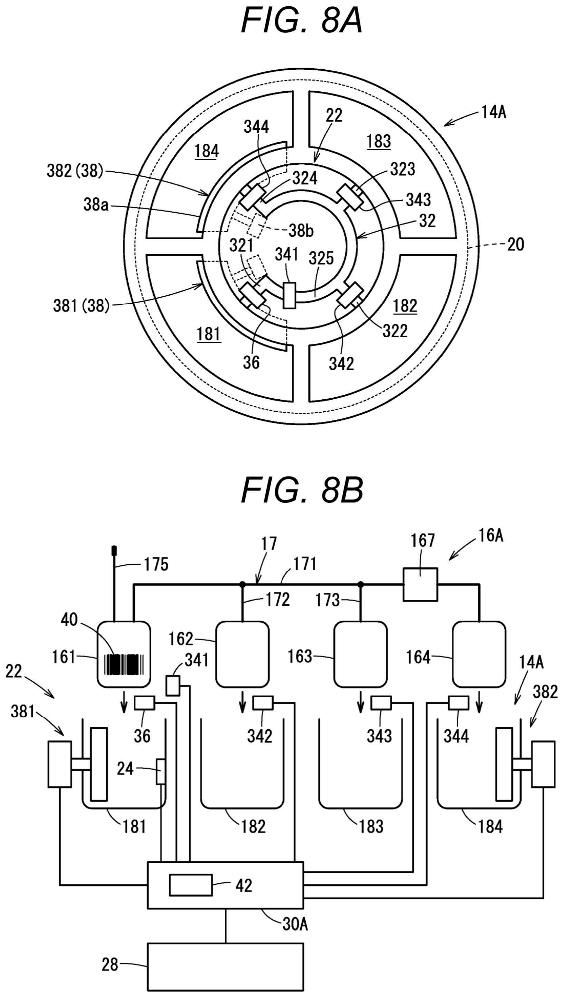

The rotating body 14A illustrated in Fig. 8A includes the first slot 181, the second slot 182, the third slot 183, and a fourth slot 184. The first slot 181 is disposed with the first extrusion unit 381, and the fourth slot 184 is disposed with the second extrusion unit 382. Note that, the third slot 183 is not disposed with the extrusion unit 38. The tube accommodation unit 32 includes a circumferential portion 325, the first flow path groove 321, the second flow path groove 322, the third flow path groove 323, and a fourth flow path groove 324. The circumferential portion 325 extends in the circumferential direction. The first flow path groove 321 is located on an inner peripheral side of the first slot 181, the second flow path groove 322 is located on an inner peripheral side of the second slot 182, the third flow path groove 323 is located on an inner peripheral side of the third slot 183, and the fourth flow path groove 324 is located on an inner peripheral side of the fourth slot 184. The first flow path groove 321, the second flow path groove 322, the third flow path groove 323, and the fourth flow path groove 324 extend shortly in a radial direction and are connected to the circumferential portion 325.

The liquid sensor 36 is disposed in the first flow path groove 321. The first close cut unit 341 is arranged in the circumferential portion 325 between the first flow path groove 321 and the second flow path groove 322. The second flow path groove 322 is disposed with the second close cut unit 342. The third flow path groove 323 is disposed with the third close cut unit 343. The fourth flow path groove 324 is disposed with a fourth close cut unit 344.

Note that, the layout of the tube accommodation unit 32 of the present embodiment is not limited to the above-described example, and various modifications can be made.

As illustrated in Fig. 8B, the rotating body 14A includes a first control unit 30A. The first control unit 30A drives the first close cut unit 341, the second close cut unit 342, the third close cut unit 343, the fourth close cut unit 344, the first extrusion unit 381, and the second extrusion unit 382 on the basis of the control signal of the centrifugal separator 12 (refer to Fig. 4).

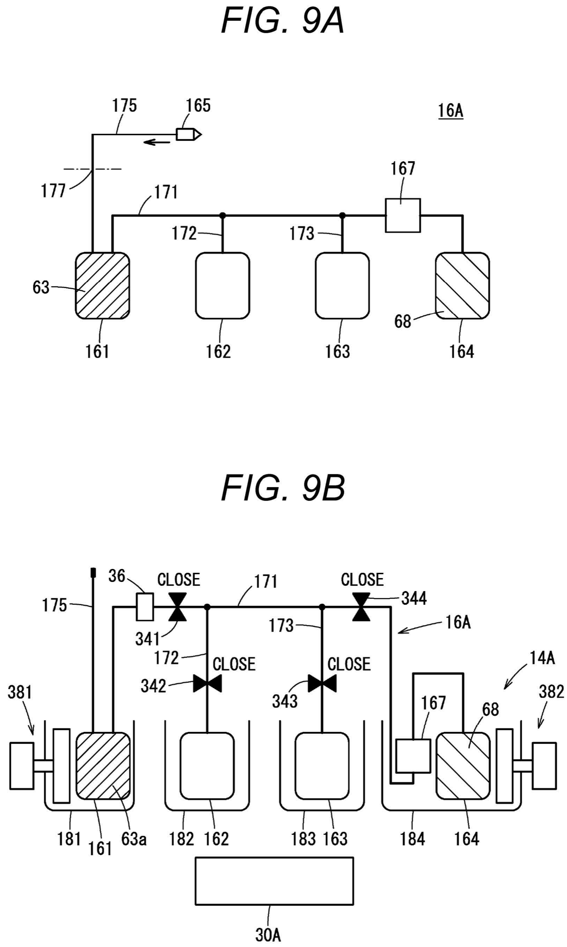

As illustrated in Fig. 9A, the centrifugal bag set 16A in an initial state includes the blood sampling needle 165, the first bag 161, the second bag 162, the third bag 163, a fourth bag 164, and the filter 167. The blood sampling needle 165 is connected to the first bag 161 via the blood sampling tube 175. The first bag 161 is an empty bag in the initial state. The first bag 161 includes the first tube 171 separately from the blood sampling tube 175. A distal end of the first tube 171 is connected to the fourth bag 164. The filter 167 is connected to the first tube 171 in the vicinity of the fourth bag 164. The filter 167 adsorbs and removes white blood cells and platelets.

The second tube 172 and the third tube 173 are connected to the first tube 171. The second bag 162 and the third bag 163 are empty bags in the initial state. The second bag 162 is connected to the first tube 171 via the second tube 172. The third bag 163 is connected to the first tube 171 via the third tube 173. The fourth bag 164 accommodates the preservative solution 68 in an initial state.

Hereinafter, processing of the blood using the rotating body 14A and the centrifugal bag set 16A of the present embodiment will be described.

As illustrated in Fig. 9A, the centrifugal bag set 16A is used for sampling the whole blood 63 by puncturing the donor with the blood sampling needle 165. The whole blood 63 sampled by the blood sampling needle 165 is accommodated in the first bag 161 through the blood sampling tube 175. The blood sampling tube 175 is cut at the illustrated cutting position 177 after the blood sampling is completed.

Next, as illustrated in Fig. 9B, the centrifugal bag set 16A is attached to the rotating body 14A. The first bag 161 of the centrifugal bag set 16A is accommodated in the first slot 181, the second bag 162 is accommodated in the second slot 182, the third bag 163 is accommodated in the third slot 183, and the fourth bag 164 is accommodated in the fourth slot 184. In the first slot 181, the identification information of the first bag 161 is read by the reading device 24. The first tube 171 is accommodated in the first flow path groove 321, the circumferential portion 325, and the fourth flow path groove 324. The liquid sensor 36, the first close cut unit 341, and the fourth close cut unit 344 are attached to the first tube 171.

The second tube 172 is accommodated in the second flow path groove 322, and the second close cut unit 342 is attached thereto. The third tube 173 is attached to the third flow path groove 323, and the third close cut unit 343 is attached thereto.

Next, the rotating body 14A is mounted on the centrifugal separator 12 in Fig. 4, and a centrifugal separation step is performed. In the centrifugal separation step, first, as illustrated in Fig. 9B, a first step of centrifugally separating the blood components 63a in the first bag 161 by rotating the rotating body 14A in a state in which the first close cut unit 341 to the fourth close cut unit 344 are closed is performed.

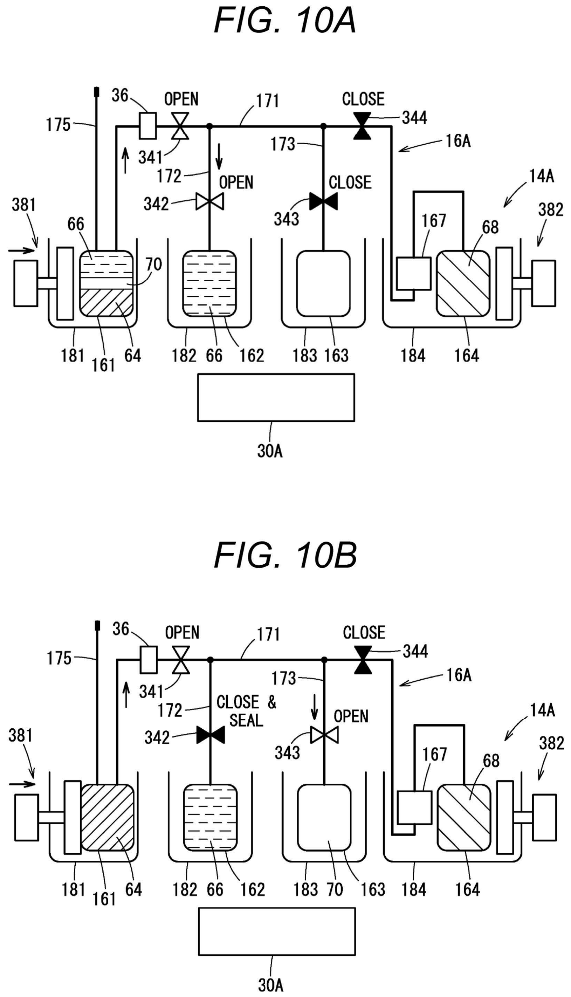

As illustrated in Fig. 10A, when the blood components 63a in the first bag 161 are centrifugally separated, they are separated into the red blood cells 64, a buffy coat 70, and the plasma 66. The plasma 66 is formed at a site close to the first tube 171, and the red blood cells 64 are formed at a position farthest from the first tube 171. The buffy coat 70 is formed between the plasma 66 and the red blood cells 64.

Next, a second step of transferring the plasma 66 to the second bag 162 is performed. At the second step, the first control unit 30A opens the first close cut unit 341 and the second close cut unit 342, and drives the first extrusion unit 381 to press the first bag 161. As a result, the plasma 66 in the first bag 161 is transferred to the second bag 162. The liquid sensor 36 detects the change in the components in the first tube 171. When the liquid sensor 36 detects a change from the plasma 66 to the buffy coat 70, the first control unit 30A closes the second close cut unit 342 and finishes the second step.

Thereafter, a third step of transferring the buffy coat 70 to the third bag 163 is performed. As illustrated in Fig. 10B, at the third step, the first control unit 30A opens the first close cut unit 341 and the third close cut unit 343. As a result, the buffy coat 70 is transferred to the third bag 163. When the liquid sensor 36 detects the change from the buffy coat 70 to the red blood cells 64, the first control unit 30A closes the third close cut unit 343 and finishes the third step.

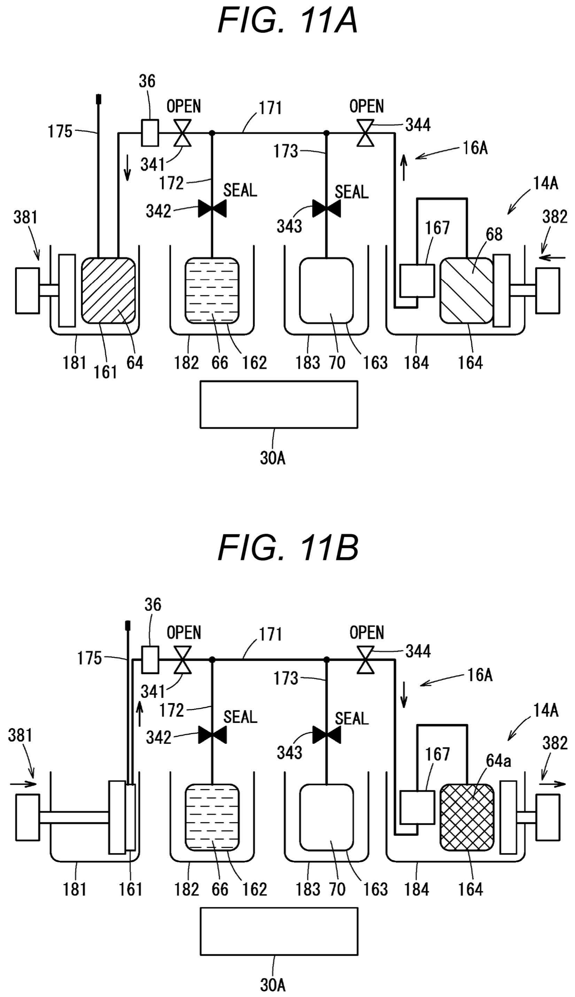

Next, as illustrated in Fig. 11A, a fourth step of transferring the preservative solution 68 in the fourth bag 164 to the first bag 161 is performed. At the fourth step, the first control unit 30A opens the first close cut unit 341 and the fourth close cut unit 344 and closes the second close cut unit 342 and the third close cut unit 343. The second extrusion unit 382 extrudes the preservative solution 68 in the fourth bag 164. As a result, the preservative solution 68 in the fourth bag 164 is transferred to the first bag 161.

Next, as illustrated in Fig. 11B, a fifth step of transferring a mixed solution 64a of the preservative solution 68 and the red blood cells 64 in the first bag 161 to the fourth bag 164 is performed. At the fifth step, the first control unit 30A presses the first bag 161 with the first extrusion unit 381. As a result, the contents of the first bag 161 are transferred to the fourth bag 164. On the way, platelets and white blood cells remaining in the mixed solution 64a in the first bag 161 are removed by the filter 167. As a result, the mixed solution 64a from which platelets and white blood cells are removed is obtained in the fourth bag 164.

The rotating body 14A of the present embodiment can perform the centrifugal separation using different types of centrifugal bag sets 16A. Conventionally, a centrifugal separator 12 dedicated to each of the centrifugal bag set 16 and the centrifugal bag set 16A has been required for the centrifugal separation processing. In contrast, since the centrifugal separation system 10 described above can use the centrifugal separator 12 common to the rotating body 14 of the first embodiment, utilization efficiency of the centrifugal separator 12 can be increased. Since different types of centrifugal separation processing can be performed only by switching between the rotating body 14 and the rotating body 14A, it is possible to perform the centrifugal separation processing which is flexible and excellent in efficiency.

### (Third Embodiment)

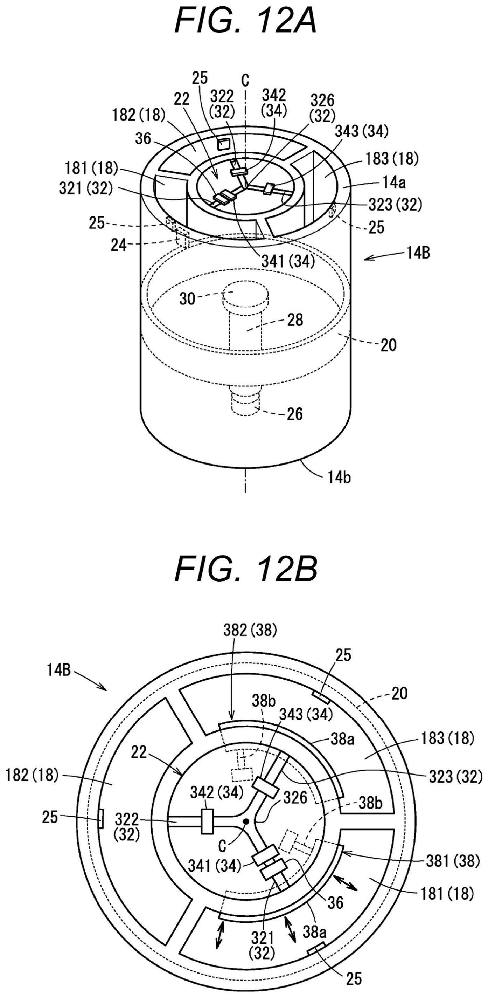

The present embodiment relates to a rotating body 14B illustrated in Figs. 12A and 12B. A basic configuration of the rotating body 14B is similar to that of the rotating body 14 (first embodiment) illustrated in Figs. 2A and 2B. In the configuration of the rotating body 14B of the present embodiment, the same reference numeral is given to the configuration similar to that of the rotating body 14 of the first embodiment, and the detailed description thereof will be omitted.

As illustrated in Figs. 12A and 12B, the rotating body 14B includes a temperature measurement unit 25 in each of the first slot 181, the second slot 182, and the third slot 183. The temperature measurement unit 25 is a contact type or non-contact type temperature sensor. The temperature measurement unit 25 disposed in the first slot 181 measures temperature of the first bag 161 accommodated in the first slot 181. The temperature measurement unit 25 disposed in the second slot 182 measures temperature of the second bag 162 accommodated in the second slot 182. The temperature measurement unit 25 disposed in the third slot 183 measures temperature of the third bag 163 accommodated in the third slot 183.

The temperature measurement unit 25 measures the temperatures of the first bag 161, the second bag 162, and the third bag 163 at regular intervals. A measurement result of the temperature measurement unit 25 is stored in the first control unit 30 while one centrifugal bag set 16 is accommodated. The measurement result of the temperature measurement unit 25 is transmitted from the first control unit 30 to the second control unit 60, and is used for operation control of the temperature regulator 54. The measurement result of the temperature measurement unit 25 is transmitted to a quality control device (not illustrated) of the blood center at a predetermined timing, and is used to confirm whether or not the first bag 161, the second bag 162, and the third bag 163 are maintained within a predetermined temperature range during the centrifugation processing. On the basis of the measurement result of the temperature measurement unit 25, the blood center transfers only the centrifugal bag set 16 maintained within the predetermined temperature range to the next step, so that the measurement result of the temperature measurement unit 25 can be used to ensure the safety of the blood preparation.

The rotating body 14B of the present embodiment makes it possible to confirm whether or not all the centrifugal bag sets 16 have been maintained at predetermined temperature during the centrifugal separation step. That is, by using the rotating body 14B of the present embodiment, it is possible to confirm that the blood preparation has been prepared within the predetermined temperature range.

Note that, the rotating body 14B of the present embodiment may be configured by attaching the temperature measurement unit 25 to each of the first slot 181, the second slot 182, the third slot 183, and the fourth slot 184 of the rotating body 14A illustrated in Fig. 8A.

Note that, the present invention is not limited to the above disclosure, and various configurations can be adopted without departing from the gist of the present invention.

## Claims

1. A centrifugal separation system that performs centrifugal separation using a centrifugal bag set including a first bag that accommodates a liquid to be processed, one or a plurality of child bags that exchanges the liquid with the first bag, and at least one tube that connects the first bag to the child bag,
the centrifugal separation system comprising:
a rotating body that accommodates the one centrifugal bag set; and
a centrifugal separator that rotates the rotating body, wherein
the rotating body is attached to and detached from the centrifugal separator that centrifugally separates the centrifugal bag set in a state in which the centrifugal bag set is accommodated.

2. The centrifugal separation system according to claim 1, wherein the rotating body includes:
a plurality of slots that is arranged to be spaced apart from each other in a circumferential direction of a rotation axis, and accommodates the first bag and the child bag to be spaced apart from each other in the circumferential direction; and
a liquid delivery mechanism that transfers the liquid between the first bag and the child bag spaced apart from each other in the circumferential direction.

3. The centrifugal separation system according to claim 2, wherein the rotating body includes a balancer that cancels a displacement in gravity center position of the centrifugal bag set.

4. The centrifugal separation system according to claim 2, wherein the liquid delivery mechanism includes an extrusion unit that presses the first bag or the child bag in at least one of the slots.

5. The centrifugal separation system according to claim 4, wherein the liquid delivery mechanism includes a tube accommodation unit that accommodates the tube, and a close cut unit that is disposed in the tube accommodation unit and is capable of closing or sealing the tube.

6. The centrifugal separation system according to claim 5, wherein the rotating body includes a liquid sensor that detects a component of the liquid inside the tube.

7. The centrifugal separation system according to claim 2, wherein the rotating body includes a reading device that reads identification information from the centrifugal bag set.

8. The centrifugal separation system according to claim 7, wherein the rotating body includes a first control unit that receives a control signal of the centrifugal separator and transmits the identification information of the centrifugal bag set to the centrifugal separator.

9. The centrifugal separation system according to claim 8, wherein the first control unit drives the liquid delivery mechanism on a basis of the control signal.

10. The centrifugal separation system according to claim 8, wherein the centrifugal separator includes:
a rotating body accommodation chamber that accommodates the rotating body;
a rotational drive unit that rotates the rotating body; and
a second control unit that controls the rotational drive unit, and
the second control unit monitors the rotating body and delivers the control signal to the first control unit.

11. The centrifugal separation system according to claim 2, wherein the rotating body includes a temperature measurement unit disposed in each of the plurality of slots, and the temperature measurement unit measures temperatures of the first bag and the child bag.

12. A rotating body of a centrifugal separation system that performs centrifugal separation using one centrifugal bag set including a first bag that accommodates a liquid to be processed, one or a plurality of child bags that exchanges the liquid with the first bag, and a tube that connects the first bag to the child bag, the rotating body comprising:
a first slot that accommodates the first bag;
at least one second slot that is arranged to be spaced apart from the first slot in a circumferential direction of a rotation axis and accommodates the child bag; and
a liquid delivery mechanism that transfers the liquid between the first bag of the first slot and the child bag of the second slot,
the rotating body being attached to and detached from a centrifugal separator that centrifugally separates the centrifugal bag set in a state in which the centrifugal bag set is accommodated.

13. The rotating body according to claim 12, further comprising: a temperature measurement unit disposed in each of the first slot and the second slot, wherein the temperature measurement unit measures temperatures of the first bag and the child bag.

14. A centrifugal separator of a centrifugal separation system that performs centrifugal separation using one centrifugal bag set including a first bag that accommodates a liquid to be processed, one or a plurality of child bags that exchanges the liquid with the first bag, and a tube that connects the first bag to the child bag, the centrifugal separator comprising:
a rotational drive unit that supports a rotating body that accommodates the centrifugal bag set so as to be attachable and detachable and rotates the rotating body; and
a second control unit that delivers a control signal to the rotating body, wherein
the second control unit monitors an operation of the rotating body.
